# EUROPEAN PATENT APPLICATION

(11) **EP 4 356 857 A1**
(43) Date of publication of application: **24.04.2024**
(21) Application number: 23204420.6
(22) Date of filing: 18.10.2023
(51) Int. Cl.: A61B 18/14, A61B 18/00, A61B 18/12, A61B 90/00

(54) **BASKET CATHETER WITH POROUS SHEATH**

(30) Priority: 19.10.2022 US 202217968901
(71) Applicant: BIOSENSE WEBSTER (ISRAEL) LTD., 2066717 Yokneam (IL)
(72) Inventor: GOVARI, Assaf, 2066717 Yokneam (IL); BEECKLER, Christopher Thomas, Irvine, 92618 (US); KEYES, Joseph Thomas, Irvine, 92618 (US); LICHTER, Justin George, Irvine, 92618 (US)
(74) Representative: Carpmaels & Ransford LLP

(57) **Abstract**

Medical apparatus includes an insertion tube configured for insertion into a body cavity of a patient and an expandable assembly connected distally to the insertion tube and comprising electrodes, which are configured to apply electrical energy to tissue within the body cavity. A flexible porous sheath is fitted over the expandable assembly and configured to contact the tissue within the body cavity so that the electrical energy is applied from the electrodes through the sheath to the tissue.

## Description

### CROSS-REFERENCE TO RELATED APPLICATION

This application claims benefit of priority to prior filed U.S. Provisional Patent Application No. 63/274,334 filed on November 1, 2021 which is hereby incorporated by reference as set forth in full herein.

### FIELD

The present invention relates generally to invasive medical equipment, and particularly to apparatus for ablating tissue within the body and methods for producing and using such apparatus.

### BACKGROUND

Cardiac arrythmias are commonly treated by ablation of myocardial tissue in order to block arrhythmogenic electrical pathways. For this purpose, a catheter is inserted through the patient's vascular system into a chamber of the heart, and an electrode or electrodes at the distal end of the catheter are brought into contact with the tissue that is to be ablated. In some cases, high-power radio-frequency (RF) electrical energy is applied to the electrodes in order to ablate the tissue thermally. Alternatively, high-voltage pulses may be applied to the electrodes in order to ablate the tissue by irreversible electroporation (IRE).

Some ablation procedures use basket catheters, in which multiple electrodes are arrayed along the spines of an expandable assembly at the distal end of the catheter. The spines bend outward to form a basket-like shape and contact tissue within a body cavity. For example, U.S. Patent Application Publication 2020/0289197 describes devices and methods for electroporation ablation therapy, with the device including a set of spines coupled to a catheter for medical ablation therapy. Each spine of the set of spines may include a set of electrodes formed on that spine. The set of spines may be configured for translation to transition between a first configuration and a second configuration.

### SUMMARY

Embodiments of the present invention that are described hereinbelow provide improved apparatus for ablating tissue with the body, as well as methods for producing and using such apparatus.

There is therefore provided, in accordance with an embodiment of the invention, medical apparatus, including an insertion tube configured for insertion into a body cavity of a patient and an expandable assembly connected distally to the insertion tube and including electrodes, which are configured to apply electrical energy to tissue within the body cavity. A flexible porous sheath is fitted over the expandable assembly and configured to contact the tissue within the body cavity so that the electrical energy is applied from the electrodes through the sheath to the tissue.

There is also provided, in accordance with an embodiment of the invention, a method for producing a medical device, which includes providing an insertion tube configured for insertion into a body cavity of a patient, and connecting distally to the insertion tube an expandable assembly including electrodes. A flexible porous sheath is fitted over the expandable assembly so that the sheath contacts tissue within the body cavity when the insertion tube is inserted into the body cavity.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
Fig. 1 is a schematic pictorial illustration showing a system for cardiac ablation, in accordance with an embodiment of the invention;
Fig. 2 is a schematic side view of a catheter expandable assembly with a porous sheath, in accordance with an embodiment of the invention;
Figs. 3A and 3B are schematic cutaway views of the catheter expandable assembly of Fig. 2 in collapsed and expanded configurations, respectively, in accordance with an embodiment of the invention;
Fig. 4 is a flow chart that schematically illustrates a method for producing a sheath for a catheter expandable assembly, in accordance with an embodiment of the invention;
Fig. 5 is a schematic side view of a system for producing sheaths for catheter basket assemblies, in accordance with an embodiment of the invention;
Fig. 6 is a schematic side view of a braided tube produced using the system of Fig. 5, in accordance with an embodiment of the invention;
Fig. 7A is a side view of an exemplary expandable member that can be used with the braided outer cover of Fig. 6, in accordance with an embodiment of the invention; and
Fig. 7B is a side view of yet another expandable member that can be used with the braided outer cover of Fig. 6, in accordance with an embodiment of the invention.

### DETAILED DESCRIPTION

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ± 10% of the recited value, e.g. "about 90%" may refer to the range of values from 81% to 99%.

In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator whereas "distal" indicates a location further away to the operator or physician.

When used herein, the terms "tubular" and "tube" are to be construed broadly and are not limited to a structure that is a right cylinder or strictly circumferential in cross-section or of a uniform cross-section throughout its length. For example, the tubular structure or system is generally illustrated as a substantially right cylindrical structure. However, the tubular system may have a tapered or curved outer surface without departing from the scope of the present invention.

Basket catheters are useful in performing ablation procedures rapidly and efficiently, because the spines of the basket catheter (and thus the electrodes on the spines) are able to contact and ablate the tissue at multiple locations concurrently. The spines themselves, however, can give rise to dangerous blood clots during the ablation procedure, due to the disturbance they cause in the blood flow, as well as due to arcing between the spines, particularly in IRE-based ablation. Furthermore, a spine can become embedded in the tissue during the procedure, which can lead to local overheating, resulting in charring and/or other trauma. The use of spines having smooth, rounded profiles can be helpful in mitigating these effects, but by itself does not eliminate the problems of clotting and tissue damage.

Embodiments of the present invention that are described herein address these problems by covering the expandable assembly with a porous sheath. As used herein, the term "sheath" is intended to include "an outer cover" or a "membrane". The sheath prevents direct contact between the spines and the tissue, while still permitting electrical energy to be applied from the electrodes through the sheath to the tissue. The type of material and thickness of the sheath may be chosen so that irrigation fluid delivered through the catheter to the expandable assembly can pass outward through the sheath to the tissue, while still preventing blood from penetrating inward through the sheath from the body cavity. The sheath is thus useful in preventing both clotting and tissue damage.

Based on these principles, the disclosed embodiments provide medical apparatus comprising an insertion tube for insertion into a body cavity of a patient and an expandable assembly connected distally to the insertion tube. A flexible porous sheath is fitted over the expandable assembly so that the sheath contacts the tissue within the body cavity. The expandable assembly comprises electrodes, which apply electrical energy through the sheath to tissue within the body cavity. Although the embodiments that are described hereinbelow relate specifically to a basket catheter for intracardiac ablation, the principles of the present invention may be adapted for use in other sorts of procedures in which electrical energy is applied to biological tissues.

In some embodiments, an electrical signal generator applies electrical energy to the electrodes on the expandable assembly with an amplitude sufficient to ablate the tissue contacted by the spines. In one embodiment, the electrical signal generator applies bipolar electrical pulses to the electrodes with an amplitude sufficient so that the electrical energy applied from the electrodes through the sheath causes irreversible electroporation (IRE) in the tissue. Additionally or alternatively, the electrical signal generator applies a radio-frequency (RF) current to the electrodes with a power sufficient so that the electrical energy applied from the electrodes through the sheath causes thermal ablation of the tissue.

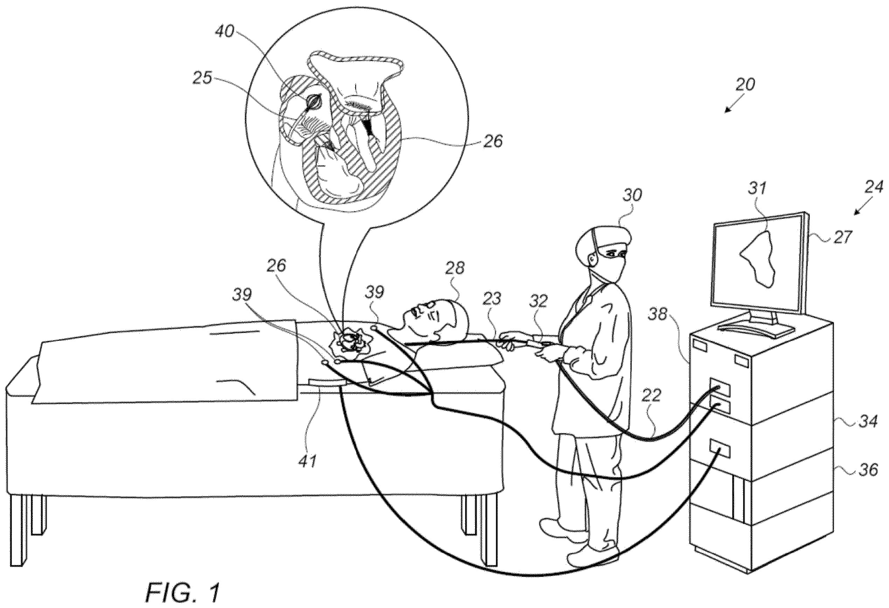

Fig. 1 is a schematic pictorial illustration of a system 20 used in an ablation procedure, in accordance with an embodiment of the invention. Elements of system 20 may be based on components of the CARTO^{®} system, produced by Biosense Webster, Inc. (Irvine, California).

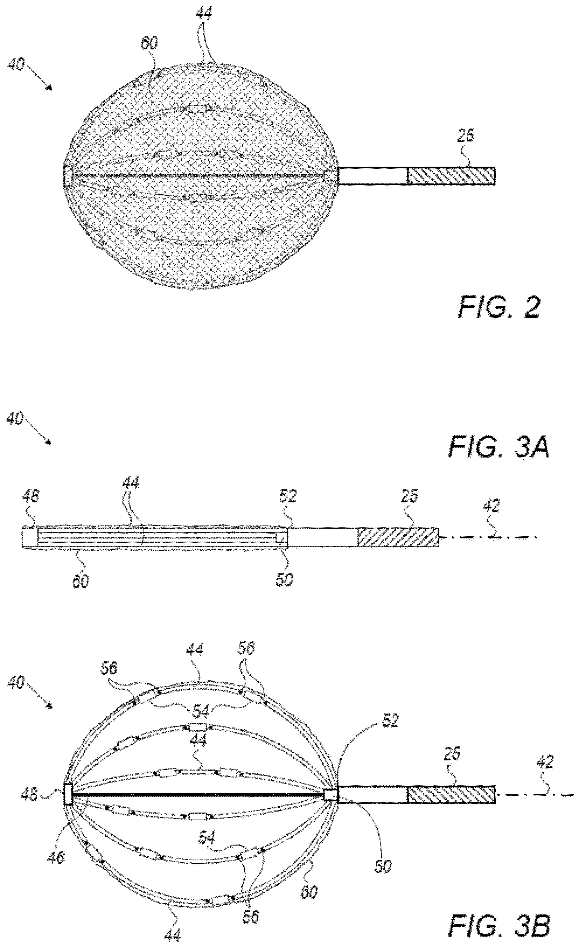

A physician 30 navigates a catheter 22 through the vascular system of a patient 28 into a chamber of a heart 26 of the patient, and then deploys an expandable assembly 40 (or 40'), over which a flexible porous sheath is fitted (as shown in detail in Fig. 2, Fig. 3A, and Fig. 3B), at the distal end of the catheter 22. The proximal end of expandable assembly 40 (or 40') is connected to the distal end of an insertion tube 25, which physician 30 steers using a manipulator 32 near the proximal end of catheter 22. Expandable assembly 40 is inserted in a collapsed configuration through a tubular delivery sheath 23, which passes through the vascular system of patient 28 into the heart chamber where the ablation procedure is to be performed. Once inserted into the heart chamber, expandable assembly 40 (or 40') is deployed from the tubular sheath and allowed to expand within the chamber. Catheter 22 is connected at its proximal end to a control console 24. A display 27 on console 24 may present a map 31 or other image of the heart chamber with an icon showing the location of expandable assembly 40 (or 40') in order to assist physician 30 in positioning the expandable assembly at the target location for the ablation procedure.

Once expandable assembly 40 (or 40') is properly deployed and positioned in heart 26, physician 30 actuates an electrical signal generator 38 in console 24 to apply electrical energy (such as IRE pulses or RF waveforms) to the electrodes on the expandable assembly, under the control of a processor 36. The electrical energy may be applied in a bipolar mode, between pairs of the electrodes on expandable assembly 40 (or 40'), or in a unipolar mode, between the electrodes on expandable assembly 40 (or 40') and a separate common electrode, for example a conductive back patch 41, which is applied to the patient's skin. During the ablation procedure, an irrigation pump 34 delivers an irrigation fluid, such as saline solution, through insertion tube 25 to expandable assembly 40 (or 40').

Typically, catheter 22 comprises one or more position sensors (not shown in the figures), which output position signals that are indicative of the position (location and orientation) of expandable assembly 40 (or 40'). For example, expandable assembly 40 (or 40') may incorporates one or more magnetic sensors, which output electrical signals in response to an applied magnetic field. Processor 36 receives and processes the signals in order to find the location and orientation coordinates of expandable assembly 40 (or 40'), using techniques that are known in the art and are implemented, for example, in the above-mentioned Carto system. Alternatively or additionally, system 20 may apply other position-sensing technologies in order to find the coordinates of expandable assembly 40 (or 40'). For example, processor 36 may sense the impedances between the electrodes on expandable assembly 40 (or 40') and body-surface electrodes 39, which are applied to the chest of patient 28, and may convert the impedances into location coordinates using techniques that are likewise known in the art. In any case, processor 36 uses the coordinates in displaying the location of expandable assembly 40 (or 40') on map 31.

Alternatively, catheter 22 and the ablation techniques that are described herein may be used without the benefit of position sensing. In such embodiments, for example, fluoroscopy and/or other imaging techniques may be used to ascertain the location of expandable assembly 40 (or 40') in heart 26.

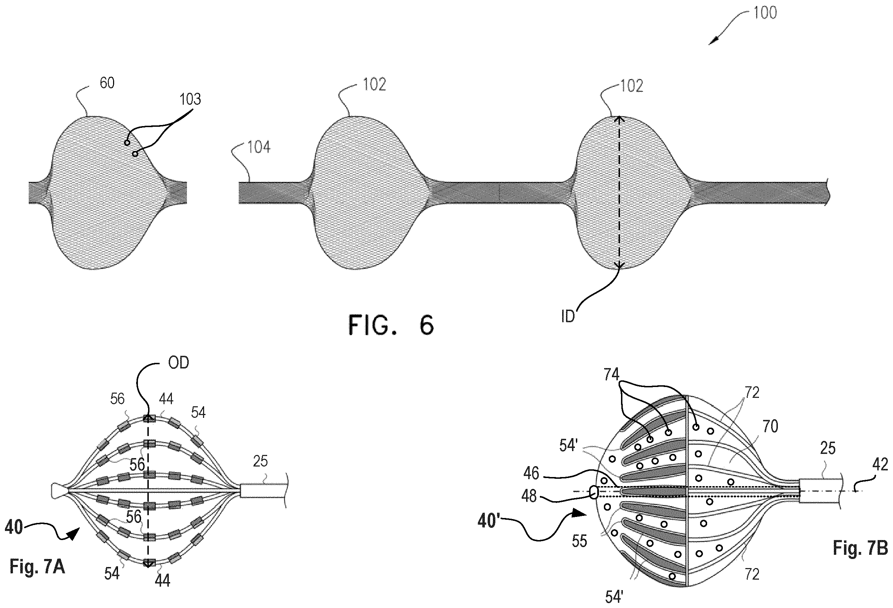

The system configuration that is shown in Fig. 1 is presented by way of example for conceptual clarity in understanding the operation of embodiments of the present invention. For the sake of simplicity, Fig. 1 shows only the elements of system 20 that are specifically related to expandable assembly 40 and ablation procedures using the expandable assembly. As used herein, the term "expandable assembly" includes either of the assembly 40 (Figs. 2, 3A, 3B, and 7A) or 40' (Fig. 7B). The remaining elements of the system will be apparent to those skilled in the art, who will likewise understand that the principles of the present invention may be implemented in other medical therapeutic systems, using other components. All such alternative implementations are considered to be within the scope of the present invention.

Reference is now made to Figs. 2, 3A and 3B, which schematically show details of expandable assembly 40, which is covered by a flexible, outer covering or porous sheath 60 in accordance with an embodiment of the invention. Fig. 2 is a side view of expandable assembly 40 in its expanded state, while Figs. 3A and 3B are cutaway views showing the expandable assembly 40 in collapsed and expanded states (with outer covering 60), respectively.

Expandable assembly 40 has a distal end 48 and a proximal end 50, which is connected to a distal end 52 of insertion tube 25. The expandable assembly comprises multiple spines 44, whose proximal ends are conjoined at proximal end 50, and whose distal ends are conjoined at distal end 48. One or more electrodes 54 are disposed externally on each of spines 44. Alternatively, spines 44 may comprise a solid conducting material and may thus serve as electrodes themselves, for example as described in U.S. Patent Application SN 16/842,648 (BIO6265USNP1) filed April 7, 2020, published as U.S. Patent Publication 2021/0307815A1 whose disclosure is incorporated herein by reference.

Irrigation outlets 56 in spines 44 allow irrigation fluid flowing within the spines 44 to exit and irrigate tissue in the vicinity of electrodes 54. Alternatively or additionally, the irrigation outlets may be located elsewhere in the expandable assembly, for example on an irrigation manifold that is contained inside the expandable assembly (not shown in the figures).

Sheath 60 is fitted over expandable assembly 40 and thus contacts the tissue in heart 26 when the expandable assembly is expanded and advanced against the tissue. Sheath 60 prevents direct contact between spines 44 and the heart tissue. Thus, the electrical energy that is applied to electrodes 54 passes through sheath 60 to the tissue. In one embodiment, sheath 60 comprises expanded polytetrafluoroethylene (ePTFE), for example with a thickness of about 70 µm. The ePTFE sheath is advantageous in being lubricious, smooth, strong, and biocompatible and in preventing spines 44 from becoming embedded in the heart tissue.

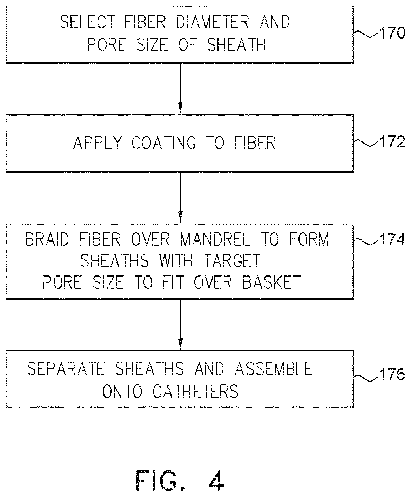

Alternatively, sheath 60 comprises a tube made by braiding suitable polymer fibers, such as a polyethylene terephthalate (PET) or polyamide (nylon) yarn. The tube may be braided with a variable diameter so as to conform better to the deployed basket shape. Specifically, the proximal diameter of the tube may be made to fit the proximal neck of basket, and the distal diameter may be made as small as possible. The distal end may be closed by fastening the loose yarn ends with an adhesive, melting the yarn ends together, or any other suitable method of sealing. An advantage of utilizing a fabric in a tubular shape rather than a flat shape is that the material better conforms to the basket shape, and pleats are avoided or minimized. Avoidance of pleats is helpful in reducing the collapsed diameter of sheath 60 and also reduces the potential for blood to coagulate in the folds of the material. A process for production of this sort of braided sheath is described further hereinbelow with reference to Figs. 4-6.

In yet another embodiment, sheath 60 is made from a sheet of flexible, non-porous material, and pores of the desired size are drilled through the material, for example by laser drilling. In yet a further embodiment the sheath 60 can be formed by blow molding a smaller tubular member to form a balloon membrane with pores subsequently formed through the balloon membrane via laser drilling.

The pores in sheath 60 are sufficiently large to permit the irrigation fluid to pass from irrigation outlets 56 outward through sheath 60 to irrigate the heart tissue, while preventing blood from penetrating inward through the sheath from the heart chamber. The inventors have found it advantageous for this purpose that the pores 103 (Fig. 6) in the sheath 60 have pore areas from approximately 10 µm² to approximately 100,000 µm². The best results were obtained with pores having areas from approximately 100 µm² to approximately 10,000 µm². These ranges of pore areas 103 are also useful in ensuring that irrigation fluid (which is electroconductive) can flow from inside the sheath 60 through the pore areas 103 and outside the porous membrane, sheath 60 to allow the electrical energy from the electrodes 54 to pass freely out through the sheath 60 to the adjoining tissue in order to ablate the tissue.

The polymer fibers that are used in producing sheath 60, such as PET and nylon fibers, are inherently insulators. Both PET and nylon, however, are hygroscopic, and once the fibers absorb water or irrigation fluid, they become more conductive and thus enable the electrical energy output by electrodes 54 to pass more freely through the sheath 60 to the target tissue. To enhance the performance of the sheath 60 in this respect, in one embodiment the polymer fibers are coated with a hydrophilic material. The hydrophilic coating attracts water into the fibers, so that sheath 60 becomes more conductive and thus facilitates efficient ablation. The coating also makes the sheath more lubricious, so that blood cells do not adhere to the fibers of the sheath.

In an alternative embodiment, a hydrophobic coating is applied to the polymer fibers of the sheath 60. The hydrophobic coating requires the sheath 60 to be pressurized in order for irrigation fluid to flow through it. This positive pressure prevents blood from entering the sheath 60 even when the irrigation is at a low flow rate.

In the collapsed state of Fig. 3A, spines 44 are straight and aligned parallel to a longitudinal axis 42 of insertion tube 25, to facilitate insertion of expandable assembly 40 into heart 26. In this state, sheath 60 collapses inward together with the spines 44. To ensure that the sheath 60 can collapse with the expandable assembly 40, the sheath 60 is joined at the distal end 48 and proximal end 50 of the expandable assembly 40. Upon extension of actuator 46 to separate the distal end 48 and proximal end 50 both the sheath 60 and the spines 44 will compress into a tubular profile of Fig. 3A. Upon retraction of actuator towards the proximal end 50, the spines 44 and the sheath 60 will expand into the spherical like configuration shown in Fig. 3B. In the expanded state of Fig. 3B, spines 44 bow radially outward, causing sheath 60 to expand and contact tissue within the heart.

In one embodiment, spines 44 are produced such that the stable state of expandable assembly 40 is the collapsed state of Fig. 3A. In this case, when expandable assembly 40 is pushed out of the sheath, it is expanded by drawing an actuator 46, such as a suitable wire, in the proximal direction through insertion tube 25. Releasing actuator 46 allows expandable assembly 40 to collapse back to its collapsed state.

In another embodiment, spines 44 are produced such that the stable state of expandable assembly 40 is the expanded state of Fig. 3B. In this case, expandable assembly 40 opens out into the expanded stated when it is pushed out of the sheath, and actuator 46 may be replaced by a flexible pusher rod for straightening spines 44 before withdrawing the expandable assembly back into the sheath.

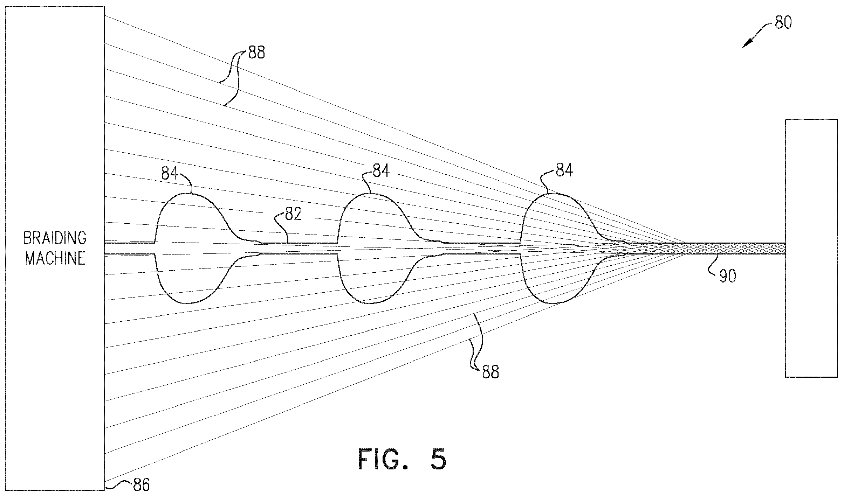

Reference is now made to Figs. 4-6, 7A and 7B which schematically illustrate a method for producing sheaths 60 for a catheter expandable assembly, in accordance with an embodiment of the invention. Fig. 4 is a flow chart showing steps in the method, while Fig. 5 is a schematic side view of a system 80 for producing the sheaths. Fig. 6 is a schematic side view of a braided tube 100 produced using the system of Fig. 5.

As a preliminary step, the diameter of fibers 88 that are to be used in producing the sheaths and the sizes of the pores to be formed in the sheaths are selected, at a fiber selection step 170. For example, PET or nylon fibers of approximately 25 to 100 denier may be used, and the pores in the sheath may have areas from approximately 10 µm² to approximately 100,000 µm², as noted above. If desired, a hydrophilic or hydrophobic coating may be applied to the fibers, at a coating step 172.

Fibers 88 are braided over a suitable mandrel 90 to form a tube 100 having a varying diameter, at a braiding step 174. As shown in Fig. 5, mandrel 90 comprises multiple bulbous protrusions 84 disposed along a shaft 82. The bulbous protrusion 84 can be a balloon member inflated to a desired shape so that it serves as underlying support structure for the braiding of the fibers 88. A braiding machine 86, as is known in the art, braids fibers 88 over mandrel 90. The resulting tube 100, as shown in Fig. 6, comprises bulbs 102 of the desired size, with narrower necks 104 in between. Bulbs 102 are sized to fit over basket assemblies 40, while necks 104 fit snugly over the distal end of insertion tube 25 (as shown in Fig. 2). The braiding parameters of braiding machine 86 are set so that bulbs 102 contain openings or pores 103 of the desired size (e.g., pore diameters or pore areas). To ensure firm contact between the braided outer cover 60 and the expandable assembly 40, the braided outer cover 60 can be sized to be slightly smaller than the expandable assembly 40. For example, each bulb 84 (defining the inside diameter of outer cover 60) can be sized such that the maximum outer diameter of the bulbous protrusions 84 of mandrel 90 (which is also the maximum inside diameter ID of bulb 102) is approximately 5% to 20% smaller than the maximum outer OD diameter of the expandable assembly 40 or 40' (Fig. 7A and 7B). The maximum outside diameter OD of the expandable assembly 40 can be measured from the radially outermost points of the expandable assembly (e.g., from one electrode to diametrically opposed electrode (Fig. 7A) or from one spine to a diametrically opposed spine).

Necks 104 in tube 100 are cut to separate the tube 100 into separate multiple bulbs 102, which can now be considered to be sheaths 60, at a sheath separation step 176. It is noted that prior to the separation of bulbs 102 into individual members or sheaths 60, the underlying balloons or bulbous members 84 of mandrel 90 are deflated and withdrawn through tube 100. Alternatively, after separation of bulbs 102 into separate pieces, the underlying balloon or bulbous member 84 can also be withdraw at this stage. As noted earlier, the distal ends of bulbs 102 are closed after cutting by fastening together the loose ends of fibers 88 with an adhesive, melting the ends together, or any other suitable method of sealing. The sheaths 60 (formerly bulbs 102) are then fitted over basket assemblies 40 or 40' by compressing expandable assembly 40 (Fig. 7A) or deflating assembly 40' (Fig. 7B) so that assembly 40 or 40' will fit within the smaller tubular member connecting the sheath 60 (e.g., neck 104).

In the embodiment of Fig. 7B, the expandable assembly 40' is in the form of a balloon membrane 70 coupled to a distal end of the tubular shaft 25. A plurality of electrodes 54' can be disposed on respective substrates 55 disposed on the outer surface of the membrane 70. Conductive members 72 can be used to deliver electrical energy to respective electrodes 54'. Conductive members 72 can be disposed inside or outside the membrane 70 in the form of electrical traces. Alternatively, conductive members 72 can be wires disposed within the internal volume defined by balloon membrane 70. Conductive members 72 can extend through the insertion tube 25 all the way to the ablation generator. Irrigation pores 74 extend through balloon membrane 70 to allow irrigation fluid delivered from insertion tube 25 to flow through membrane 70 and through the pores 103 of the outer porous covering 60. An actuator 46 can be mounted inside the membrane 70 (and shown as dashed lines) so that actuator is fixed to the distal hub 48 and allow for extension of hub 48 relative to insertion tube 25 (i.e., compressing the membrane 70 into a smaller outer profile) or retraction of hub 48 relative to insertion tube 25 (i.e., causing expansion of membrane 70 into a larger profile). Membrane 70 is preferably made of a less flexible material than porous covering 60.

Assembly of expandable member 40' can be completed by deflating the membrane 70 and inserting the member 40' into the smaller tube (e.g., neck 104) of sheath 60. Thereafter, the member 40' can be inflated and the ends of the sheath 60 can be joined to the proximal and distal end of membrane 70. Details for an embodiment of the expandable member 40' can be understood with reference to U.S. Patent Application SN 16/707,175 (BIO6195USNP1) filed December 9, 2019, published as U.S. Patent Publication 2021/0169567A1 which is hereby incorporated by reference as if set forth herein.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

## Claims

1. Medical apparatus, comprising:
an insertion tube configured for insertion into a body cavity of a patient;
an expandable assembly connected distally to the insertion tube and comprising electrodes, which are configured to apply electrical energy to tissue within the body cavity; and
a flexible porous sheath, which is fitted over the expandable assembly and configured to contact both the tissue within the body cavity and the electrodes so that the electrical energy is applied from the electrodes through the sheath to the tissue.

2. The apparatus according to claim 1, wherein the sheath comprises a braided polymer fiber, optionally wherein the sheath is braided as a tube of varying diameter.

3. The apparatus according to claim 1, wherein the sheath comprises a polymer fiber having a hydrophilic coating.

4. The apparatus according to claim 1, wherein the expandable assembly comprises one or more irrigation outlets, which are coupled to convey an irrigation fluid from the insertion tube to the tissue through the sheath.

5. The apparatus according to claim 4, wherein the sheath comprises a fabric chosen to permit the irrigation fluid to pass outward through the sheath from the one or more irrigation outlets to the tissue while preventing blood from penetrating inward through the sheath from the body cavity.

6. The apparatus according to claim 1, wherein the porous sheath contains pores having respective areas between 10 µm² and 100,000 µm², optionally wherein the respective areas of the pores are between 100 µm² and 10,000 µm².

7. The apparatus according to claim 1, wherein the expandable assembly comprises a plurality of resilient spines, having respective proximal and distal tips, wherein the proximal tips of the spines are joined mechanically at a proximal end of the expandable assembly, and the distal tips of the spines are joined mechanically at a distal end of the expandable assembly, and the spines bow radially outward against the sheath when the expandable assembly is deployed in the body cavity, thereby causing the sheath to contact the tissue in the body cavity.

8. The apparatus according to claim 1, wherein the expandable assembly comprises
a balloon membrane that includes a plurality of electrodes disposed radially on an outer surface of the balloon membrane and in contact with the outer porous sheath, each of the plurality of electrodes being electrically connected to at least one respective conductive member extending through the insertion tube; and
irrigation pores that extend through the balloon membrane to allow irrigation fluid to flow from the insertion tube through the irrigation pores.

9. A method for producing a medical device, comprising:
providing an insertion tube configured for insertion into a body cavity of a patient;
connecting distally to the insertion tube an expandable assembly comprising electrodes; and
fitting a flexible porous sheath over the expandable assembly so that the sheath contacts tissue within the body cavity when the insertion tube is inserted into the body cavity.

10. The apparatus according to claim 1 or the method according to claim 9, wherein the sheath comprises expanded polytetrafluoroethylene (ePTFE).

11. The method according to claim 9, wherein fitting the flexible porous sheath comprises braiding a polymer fiber to form the sheath, optionally wherein braiding the polymer fiber comprises braiding a tube with a varying diameter.

12. The method according to claim 11, and comprising applying a hydrophilic coating to the polymer fiber.

13. The method according to claim 12, wherein the porous sheath contains pores having respective areas between 10 µm² and 100,000 µm², and optionally comprising conveying an irrigation fluid from the insertion tube to the tissue through the sheath.

14. The method according to claim 9, wherein connecting the expandable assembly comprises joining together respective distal tips of a plurality of resilient spines at a proximal end of the expandable assembly, and joining together respective distal tips of the spines at a distal end of the expandable assembly, so that the spines bow radially outward when the expandable assembly is deployed in the body cavity, thereby causing the sheath to contact the tissue in the body cavity.

15. The method according to claim 9, and comprising coupling an electrical signal generator to apply electrical energy from the electrodes through the sheath to the tissue within the body cavity.
